# EUROPEAN PATENT APPLICATION

(11) **EP 4 682 519 A1**
(43) Date of publication of application: **21.01.2026**
(21) Application number: 25185496.4
(22) Date of filing: 26.06.2025
(51) Int. Cl.: G01N 25/18

(54) **THERMAL CONDUCTIVITY SENSOR FOR DETECTING A GAS**

(30) Priority: 18.07.2024 US 202418777140
(71) Applicant: Life Safety Distribution GmbH, 1180 Rolle (CH)
(72) Inventor: BECK, Scott Edward, Charlotte 28202 (US); WANG, Yong-Fa Alan, Charlotte 28202 (US); FOSTER, Philip C., Charlotte 28202 (US); PRATT, Keith Francis Edwin, Charlotte 28202 (US)
(74) Representative: Haseltine Lake Kempner LLP

(57) **Abstract**

A thermal conductivity sensor is disclosed. The thermal conductivity sensor comprises a first portion having at least one heating element and a second portion having at least one sensing element. The first portion and the second portion are positioned such that the at least one heating element and the at least one sensing element are separated by a gas channel between the first portion and the second portion that is configured to allow gas to pass through the gas channel such that the gas passes between the at least one heating element and the at least one sensing element. The at least one sensing element is configured to measure a change in temperature of the gas to detect a presence of the gas having a higher thermal conductivity.

## Description

### TECHNOLOGICAL FIELD

The present invention relates to hydrogen sensors, and more particularly relates to a thermal conductivity sensor for detecting a gas.

### BACKGROUND

Hydrogen sensors are integral components in thermal conductivity measurements, playing multifaceted roles in ensuring accuracy, safety, and control within experimental setups involving hydrogen gas. Conventional thermal conductivity sensors function by generating heat that raises the temperature of the gas surrounding the sensor and measures the temperature. When the gas contains a contaminant, such as hydrogen, that has a higher thermal conductivity than the bulk gas, the higher thermal conductivity of the contaminant gas results in more heat loss and a lower temperature. The reduction in temperature is then measured to determine the concentration of the gas in surrounding environment. However, the accuracy of the current thermal conductivity sensors can be compromised due to mismanaged heat distribution within the assembly of the thermal conductivity sensors. Further, the conventional thermal conductivity sensors have inadequate heat dissipation or inefficient insulation that can lead to temperature fluctuations. Such temperature fluctuations affect the performance of both the heating element and the sensing element, and thus result in inaccurate readings.

The inventors have identified numerous areas of improvement in the existing technologies and processes, which are the subjects of embodiments described herein. Through applied effort, ingenuity, and innovation, many of these deficiencies, challenges, and problems have been solved by developing solutions that are included in embodiments of the present disclosure, some examples of which are described in detail herein.

### BRIEF SUMMARY

The following presents a simplified summary in order to provide a basic understanding of some aspects of the present disclosure. This summary is not an extensive overview and is intended to neither identify key or critical elements nor delineate the scope of such elements. Its purpose is to present some concepts of the described features in a simplified form as a prelude to the more detailed description that is presented later.

In one example embodiment, a thermal conductivity sensor for detecting a gas is disclosed. The thermal conductivity sensor comprises a first portion having at least one heating element and a second portion having at least one sensing element. The first portion and the second portion are positioned such that the at least one heating element and the at least one sensing element are separated by a gas channel or gap between the first portion and the second portion that is configured to allow gas to pass through the gas channel or gap such that the gas passes between the at least one heating element and the at least one sensing element. The at least one sensing element is configured to measure a change in temperature of the gas to detect a presence of the gas having a higher thermal conductivity.

In some embodiments, the first portion and the second portion of the thermal conductivity sensor are arranged in a plurality of orientations. Further, the plurality of orientations comprises at least one of a vertical orientation and a horizontal orientation.

In some embodiments, in the vertical orientation, the at least one heating element is positioned over or under the at least one sensing element and separated via the gas channel such that the gas passes around the at least one heating element and the at least one sensing element.

In some embodiments, in the horizontal orientation, the at least one heating element is positioned beside the at least one sensing element and the at least one heating element and the at least one sensing element are positioned on respective elevated structures separated via the gap such that the gas passes around the at least one heating element and the at least one sensing element. In some embodiments, the gas passes above and below the at least one heating element and the at least one sensing element.

In some embodiments, the at least one sensing element is configured to measure the change in temperature of the gas to monitor heat transfer within the gas around the thermal conductivity sensor. Further, a change in the heat transfer within the gas around the thermal conductivity sensor corresponds to a change in a thermal property of the gas.

In some embodiments, in the vertical orientation, the first portion corresponds to a top cap and the second portion corresponds to a base. Further, each end of the top cap is coupled with a corresponding end of the base via an adhesive such that the gas channel is provided between the top cap and the base. The adhesive comprises at least one of a frit bond, an anodic bond, an epoxy adhesive, and a eutectic bond. The adhesive is dependent on the materials of the top cap and the base.

In some embodiments, the top cap comprises a set of vents. The set of vents are configured to allow diffusion of the gas into and through the gas channel of the thermal conductivity sensor. The top cap and the base are made from one or more materials comprising at least one of silicon, glass, or plastic.

In some embodiments, the thermal conductivity sensor further comprising an ambient temperature sensing element configured to measure the temperature of the gas passing through the gas channel in a real time.

In some embodiments, the at least one sensing element comprises at least one of a resistor, a diode, or a thermopile. In some embodiments, the at least one heating element is made from a group of materials. The group of materials comprise at least one of an Iron-Nickel (NiFe)/Permalloy, Platinum (Pt), Chromium (Cr), doped Silicon (Si) or Polysilicon, Nichrome (NiCr), Nickel (Ni), Platinum Silicide (PtSi) and other metal silicides, Tungsten (W), Titanium Nitride (TiN), Aluminum Nitride (AlN), Tungsten Nitride (WN), or any combination thereof. In some embodiments, the at least one sensing element is made from a group of materials. The group of materials comprise at least one of the NiFe/Permalloy, Pt, Cr, doped Si or polysilicon, NiCr, Ni, PtSi and other metal silicides, W, TiN, AlN, WN, or any combination thereof.

In another example embodiment, a method is disclosed. The method comprising positioning a first portion of a thermal conductivity sensor, having at least one heating element and a second portion of the thermal conductivity sensor having at least one sensing element, such that the at least one heating element and the at least one sensing element are separated by a gas channel or gap between the first portion and the second portion that is configured to allow gas to pass through the gas channel or gap such that the gas passes between the at least one heating element and the at least one sensing element. The at least one sensing element is configured to measure a change in temperature of the gas to detect a presence of the gas having a higher thermal conductivity.

The above summary is provided merely for purposes of summarizing some example embodiments to provide a basic understanding of some aspects of the invention. Accordingly, it will be appreciated that the above-described embodiments are merely examples and should not be construed to narrow the scope or spirit of the invention in any way. It will be appreciated that the scope of the invention encompasses many potential embodiments in addition to those here summarized, some of which will be further described below.

### BRIEF DESCRIPTION OF THE DRAWINGS

Having thus described certain example embodiments of the present disclosure in general terms, reference will hereinafter be made to the accompanying drawings, which are not necessarily drawn to scale, and wherein:
FIG. 1 illustrates a side sectional view of a thermal conductivity sensor in a vertical orientation in accordance with an example embodiment of the present disclosure;
FIG. 2 illustrates a top view of the thermal conductivity sensor in the vertical orientation in accordance with an example embodiment of the present disclosure;
FIG. 3A illustrates a side sectional view of a thermal conductivity sensor in a horizontal orientation in accordance with an example embodiment of the present disclosure;
FIG. 3B illustrates a top view of the thermal conductivity sensor in the horizontal orientation in accordance with an example embodiment of the present disclosure;
FIG. 4A illustrates a side sectional view of another thermal conductivity sensor in a horizontal orientation in accordance with an example embodiment of the present disclosure;
FIG. 4B illustrates a filter media associated with the another thermal conductivity sensor in the horizontal orientation in accordance with an example embodiment of the present disclosure;
FIG. 5 illustrates a graphical representation of a simulation of the thermal conductivity sensor in the vertical orientation in accordance with an example embodiment of the present disclosure;
FIG. 6 illustrates a graphical representation of effect of temperature on at least one sensing element in accordance with an example embodiment of the present disclosure;
FIG. 7 illustrates a graphical representation of a change in sensing temperature corresponding to 1% change in thermal conductivity of the gas in accordance with an example embodiment of the present disclosure;
FIG. 8 illustrates a graphical representation of a parallel plate preliminary simulation of the changes in sensing temperature due to changes in thermal conductivity sensor in accordance with an example embodiment of the present disclosure; and
FIG. 9 illustrates a graphical representation of another parallel plate preliminary simulation of the thermal conductivity sensor in accordance with an example embodiment of the present disclosure.

### DETAILED DESCRIPTION

Some embodiments will now be described more fully hereinafter with reference to the accompanying drawings, in which some, but not all, embodiments are shown. Indeed, various embodiments may be embodied in many different forms and should not be construed as limited to the embodiments set forth herein; rather, these embodiments are provided so that this disclosure will satisfy applicable legal requirements.

The components illustrated in the figures represent components that may or may not be present in various embodiments of the invention described herein such that embodiments may include fewer or more components than those shown in the figures while not departing from the scope of the invention. Some components may be omitted from one or more figures or shown in dashed line for visibility of the underlying components.

The present disclosure provides various embodiments of a thermal conductivity sensor. Embodiments may comprise a first portion having at least one heating element, and a second portion having at least one sensing element. In various embodiments, the first portion and the second portion are positioned such that the at least one heating element and the at least one sensing element are separated by a gas channel between the first portion and the second portion. In various embodiments, the gas channel is configured to allow gas to pass through the gas channel such that the gas passes between the at least one heating element and the at least one sensing element. In various embodiments, the at least one sensing element is configured to measure a change in temperature of the gas to detect a presence in the gas having a higher thermal conductivity.

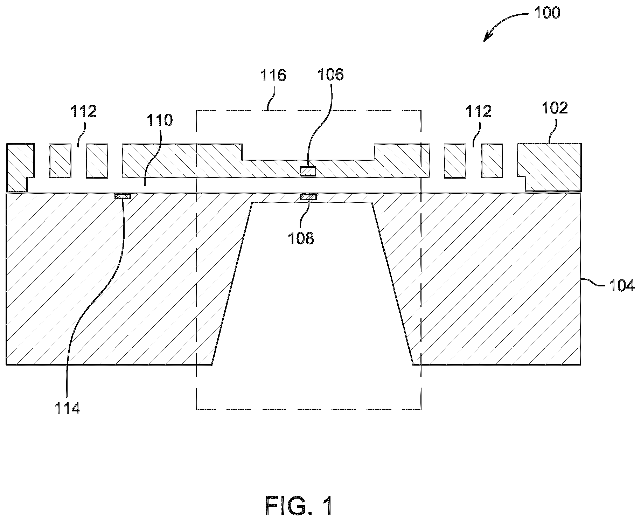

FIG. 1 illustrates a side sectional view of a thermal conductivity sensor 100 in a vertical orientation in accordance with an example embodiment of the present disclosure. The thermal conductivity sensor 100 may comprise a first portion 102 and a second portion 104.

In some embodiments, the first portion 102 may comprise at least one heating element 106. The at least one heating element 106 may be configured to heat a gas flowing through the thermal conductivity sensor 100. The first portion 102 may correspond to a thick membrane having the at least one heating element 106. In some embodiments, the thick membrane may facilitate to improve reliability of the first portion 102 of the thermal conductivity sensor 100 in an instance in which the at least one heating element 106 is heated. In some embodiments, the at least one heating element 106 may be made from a group of materials. In one example, the group of materials may comprise at least one of a Permalloy (81:19 Iron-Nickel (NiFe)) and 60:40 NiFe, preferably. The thin film of Permalloy may have temperature coefficients of resistance between 3600 parts per million per degree Celsius (ppm/° C) and 4100 ppm/° C. In another example, the group of materials may comprise at least one of Platinum (Pt), Chromium (Cr), doped Silicon (Si) or Polysilicon, Nichrome (NiCr), Nickel (Ni), Platinum Silicide (PtSi) and other metal silicides, Tungsten (W), Titanium Nitride (TiN), Aluminum Nitride (AlN), Tungsten Nitride (WN), or any combination thereof.

In some embodiments, the second portion 104 may comprise at least one sensing element 108. The at least one sensing element 108 may be configured to measure a change in temperature of the gas to detect a presence of the gas having a higher thermal conductivity. In one example, the gas may correspond to a hydrogen gas. Further, the at least one sensing element 108 may be configured to measure the change in temperature of the gas to monitor heat transfer within the gas around the thermal conductivity sensor 100. In one embodiment, a change in the heat transfer within the gas around the thermal conductivity sensor 100 may correspond to a change in a thermal property of the gas. Further, the at least one sensing element 108 may be configured to measure the change in temperature of the gas in a range corresponding to a sensing area 116. Within the sensing area 116, the at least one sensing element 108 may be configured to monitor heat transfer within the gas around the thermal conductivity sensor 100.

In some embodiments, the at least one sensing element 108 may comprise at least one of a resistor, a diode, or a thermopile. In some embodiments, the at least one sensing element 108 may be made from a group of materials or a pair of materials from the group of materials. In one example, the group of materials for a thermocouple may comprise at least one of Chromium (Cr) and Permalloy (80/20 NiFe), or Cr and 60/40 NiFe, preferably. In another thermocouple example, the group of materials may comprise at least one of polysilicon and aluminum (Al); n-type polysilicon and p-type polysilicon; Ni-Fe alloy and Chromium Disilicide; Chromium Nitride and Copper (Cu); Chromium Nitride and Al; Chromium Nitride and p-type polysilicon; and Copper (Cu) and Cu-Ni alloy. In one example embodiment, a pair of materials form thermocouples. In one example embodiment, the resistor may be made from a material from the group of materials described above. In another example embodiment, the resistor may be made from the group of materials such as platinum (Pt), Nichrome (NiCr), Platinum Silicide (PtSi) other metal silicides, tungsten (W), and other materials known in the art. Furthermore, the diode may correspond either to a p-n junction or a metal-semiconductor junction.

In some embodiments, the first portion 102 and the second portion 104 of the thermal conductivity sensor 100 may be positioned such that the at least one heating element 106 and the at least one sensing element 108 are separated by a gas channel 110 between the first portion 102 and the second portion 104. The gas channel 110 may be configured to allow gas to pass through the gas channel 110 such that the gas passes between the at least one heating element 106 and the at least one sensing element 108. In some embodiments, the at least one heating element 106 may be separated from the at least one sensing element 108 such that the at least one heating element 106 and the at least one sensing element 108 are on opposite sides of the gas channel 110. Therefore, the at least one sensing element 108 may not be heated by the at least one heating element 106 to an elevated temperature of the at least one heating element 106, and instead, directly heated by the gas.

In some embodiments, the first portion 102 and the second portion 104 of the thermal conductivity sensor 100 may be arranged in a plurality of orientations. Further, the plurality of orientations may comprise at least the vertical orientation. In some embodiments, in the vertical orientation, the at least one heating element 106 may be positioned over the at least one sensing element 108 and separated via the gas channel 110 such that the gas passes around the at least one heating element 106 and the at least one sensing element 108. In one example, the gas passes around the at least one heating element 106 and the at least one sensing element 108 may correspond to that the gas passes below or above the at least one heating element 106 and the at least one sensing element 108. In some embodiments, having the at least one heating element 106 positioned over the at least one sensing element 108 with the gas between the at least one heating element 106 positioned over the at least one sensing element 108 may produce a significant sensitivity improvement in the thermal conductivity sensor 100 in terms of detecting the thermal conductivity. In some alternate embodiments, in the vertical orientation, the at least one heating element 106 may be positioned under the at least one sensing element 108 and separated via the gas channel 110.

In some embodiments, the thermal conductivity sensor 100 may rely upon the use of heat and heat transfer. Further, a temperature difference may exist between the at least one heating element 106 and the at least one sensing element 108 in the gas. Furthermore, the change in the heat transfer may exist through the gas to which the at least one heating element 106 and the at least one sensing element 108 are exposed to. Further, the change in the heat transfer may correspond to the change in the thermal property of the gas. The change in the heat transfer may change an output of the thermal conductivity sensor 100 and may be directly related to the composition of the gas.

In some embodiments, in the vertical orientation, the first portion 102 may correspond to a top cap. Further, the top cap may comprise a set of vents 112. The set of vents 112 may be configured to allow diffusion of the gas present in the gas into and through the gas channel 110. In one example embodiment, the top cap may be made from one or more materials. The one or more materials may comprise at least one of silicon, glass, or plastic. In some embodiments, the second portion 104 may correspond to a base. In one example embodiment, the base may be made from one or more materials. The one or more materials may comprise at least one of silicon preferably. Further, the one or more materials may comprise at least one of glass, or plastic. Further, each end of the top cap may be coupled with a corresponding end of the base via an adhesive such that the gas channel 110 is provided between the top cap and the base. In one example embodiment, the adhesive may comprise at least one of a frit bond, an anodic bond, an epoxy adhesive, and a eutectic bond. In one example, silicon to silicon bonds may be made with the frit bond and the eutectic bond. In another example, glass to silicon bonds may be made with the anodic bond and the frit bond. In yet another example, each of the plastic, glass and silicon may be bonded to silicon with an adhesive such as a silicone, the epoxy adhesive, or cyanoacrylate.

Further, the thermal conductivity sensor 100 may comprise an ambient temperature sensing element 114. The ambient temperature sensing element 114 may be configured to measure the temperature of the gas passing through the gas channel 110 in a real time. In some embodiments, the ambient temperature sensing element 114 and the at least one sensing element 108 may be positioned adjacent to each other. In some embodiments, the ambient temperature sensing element 114 may comprise at least one of a temperature sensing resistor, or a temperature diode. The at least one sensing element 108 and the ambient temperature sensing element 114 may be positioned in one or more combinations. The one or more combinations may comprise at least one of the temperature resistor and the resistor, the temperature diode and the diode, or the temperature resistor and the thermopile.

In some embodiments, the gas may pass through the set of vents 112 to reach the gas channel 110. Further, the at least one heating element 106 may be configured to heat the gas. Simultaneously, the at least one sensing element 108 may be configured to read the temperature difference of the gas. The temperature difference may correspond to a difference of the temperature of the gas when the gas is passed through the set of vents 112 and the temperature of the gas when the gas is heated by the at least one heating element 106. Furthermore, a change in the heat transfer may exist through the gas, due to the temperature difference. Further, the change in the heat transfer may correspond to the change in the thermal property of the gas. The change in the heat transfer may provide the thermal conductivity of the gas. Thereafter, the thermal conductivity may be directly related to the composition of the gas.

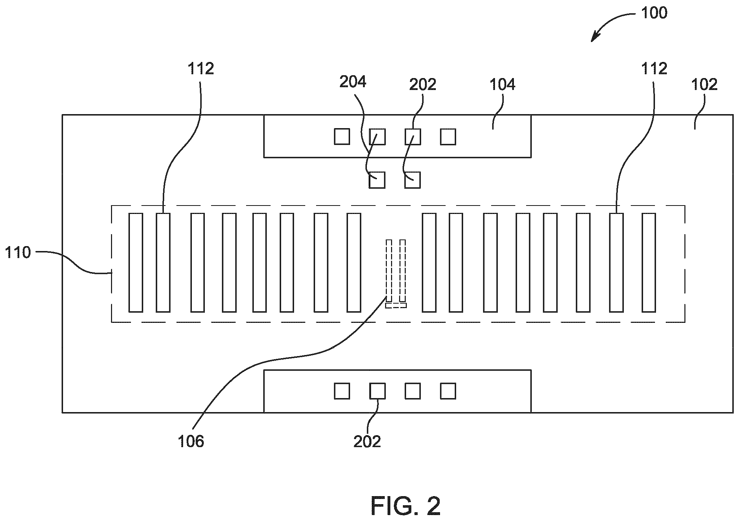

FIG. 2 illustrates a top view of the thermal conductivity sensor 100 in the vertical orientation in accordance with an example embodiment of the present disclosure. FIG. 2 is described in conjunction with FIG. 1.

The thermal conductivity sensor 100 may comprise a plurality of bond pads 202 on the first portion 102 and the second portion 104. In one example, the plurality of bond pads 202 of the first portion 102 may be bonded to the plurality of bond pads 202 on the second portion 104 via a plurality of wire bonds 204. The plurality of wire bonds 204 may link the plurality of bond pads 202 of the first portion 102 and the second portion 104, enabling the transmission of electrical signals and data across the thermal conductivity sensor 100. The linkage may ensure a seamless operation and functionality of the thermal conductivity sensor 100, as the plurality of wire bonds 204 enables the exchange of information necessary for accurate temperature measurement or control. In another example, the plurality of bond pads 202 may be bonded through silicon via, or through glass.

In some embodiments, the plurality of bond pads 202 may be isolated from the gas channel 110 by the first portion 102. The plurality of bond pads 202 may be configured to electrically connect the at least one heating element 106 with the second portion 104. The plurality of bond pads 202 may serve as points of connection for electrical signals and data transmission between the at least one heating element 106 and the second portion 104. The plurality of bond pads 202 may be isolated to safeguard the thermal conductivity sensor 100 from potential interference or contamination by the gas passing through the gas channel 110. By isolating the bond pads from the gas channel 110, the integrity and reliability of the electrical connections in the thermal conductivity sensor 100 may be preserved to minimize the risk of malfunction or damage due to environmental factors. Further, the isolation may indicate that the at least one heating element 106 may play a crucial part in the operation of the thermal conductivity sensor 100, such as facilitating precise temperature measurements or adjustments.

In some embodiments, the plurality of bond pads 202 may serve as a way the thermal conductivity sensor 100 is connected to outside of the thermal conductivity sensor 100 through other wire bonds that may then be connected to a printed circuit board (PCB) or another die. Further, electrical connection from the first portion102 to the second portion 104 may be made with through silicon or glass vias in the first portion 102. In some embodiments, the plurality of wire bonds 204 attached to the plurality of bond pads 202 may be further protected with an insulating encapsulant.

As described in FIG. 1, the first portion 102 may comprise the at least one heating element 106 and the set of vents 112. The set of vents 112 may be configured to allow diffusion of the gas present in the gas into and through the gas channel 110. The gas may enter the thermal conductivity sensor 100 from a source through the set of vents 112, initiating the process by which the gas will be heated, and/or circulated. Further, the set of vents 112 may be configured to allow diffusion of the gas out of the gas channel 110. The set of vents 112 may facilitate diffusion of the gas from within the gas channel 110 to an external environment. The allowed diffusion of gas into and out of the gas channel 110 may complete a cycle within the thermal conductivity sensor 100.

In some embodiments, the thermal conductivity sensor 100 may operate in a temperature range. The temperature range may correspond to a range of -25 degrees Celsius (° C) to 85 ° C. Further, the thermal conductivity sensor 100 may operate in a pressure range. The pressure range may correspond to a range of sea level to 12,000 feet (ft.) above the sea level. It will be apparent to one skilled in the art that the above-mentioned components of the thermal conductivity sensor 100 have been provided only for illustration purposes, without departing from the scope of the disclosure.

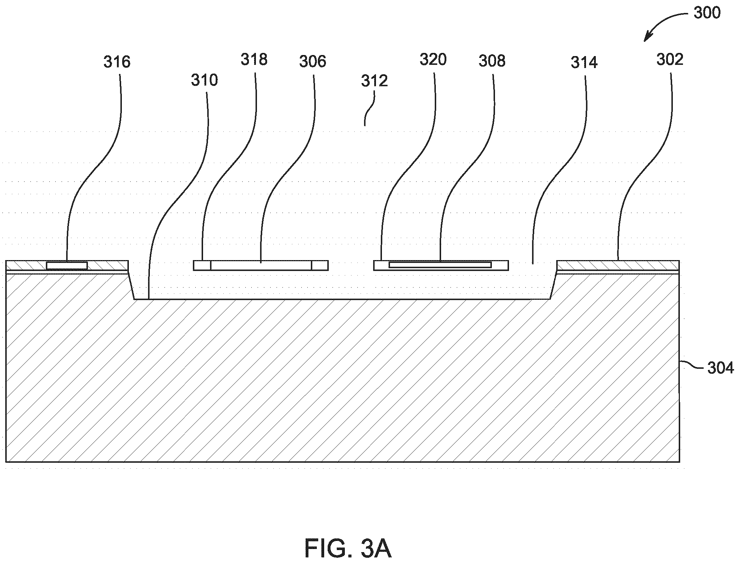

FIG. 3A illustrates a side sectional view of a thermal conductivity sensor 300 in a horizontal orientation, in accordance with an example embodiment of the present disclosure. The thermal conductivity sensor 300 may comprise a first portion 302 and a second portion 304.

In some embodiments, the first portion 302 may comprise at least one heating element 306, and at least one sensing element 308. In some embodiments, the first portion 302 may be placed on the second portion 304 having a buried cavity 310. In one example, the second portion 304 may correspond to a base. In some embodiments, the at least one heating element 306 and the at least one sensing element 308 may be positioned such that the at least one heating element 306 and the at least one sensing element 308 are separated by a gap 314 inside the buried cavity 310. The gap 314 may be configured to allow gas 312 to pass through the gap 314 such that the gas 312 passes between the at least one heating element 306 and the at least one sensing element 308, through the buried cavity 310. In some embodiments, the at least one heating element 306 may be separated from the at least one sensing element 308 such that that the gas 213 under analysis is in between the at least one heating element 306 and the at least one sensing element 308. As a result, the at least one sensing element 308 may not be heated by the at least one heating element 306 to an elevated temperature of the at least one heating element 306, and instead, directly heated by the gas 312.

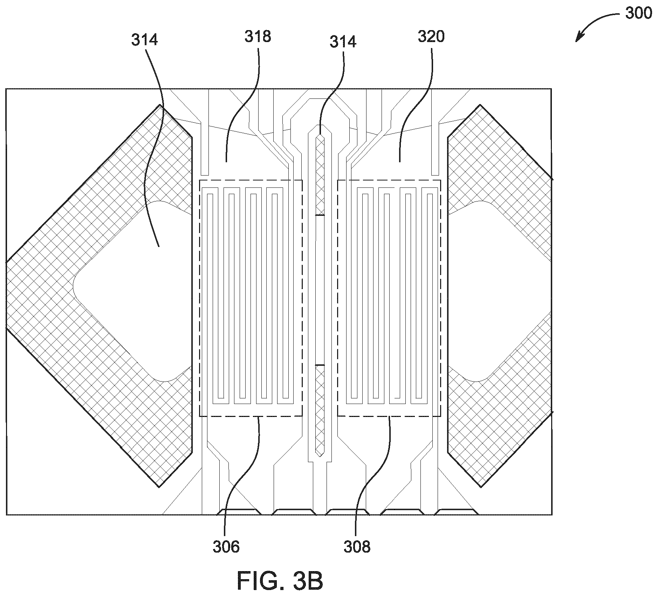

In some embodiments, the at least one heating element 306 and the at least one sensing element 308 of the thermal conductivity sensor 300 may be arranged in a plurality of orientations. Further, the plurality of orientations may comprise at least the horizontal orientation. In some embodiments, in the horizontal orientation, the at least one heating element 306 may be positioned beside the at least one sensing element 308. In one example, the at least one heating element 306 may be positioned on left side of the at least one sensing element 308. In another example, the at least one heating element 306 may be positioned on right side of the at least one sensing element 308. Further, the at least one heating element 306 and the at least one sensing element 308 may be positioned on respective elevated structures 318, 320 on the first portion 302, separated via the gap 314 such that the gas 312 passes around the at least one heating element 306 and the at least one sensing element 308. In one example, the gas 312 passes around the at least one heating element 306 and the at least one sensing element 308 may correspond to that the gas 312 passes above or below the at least one heating element 306 and the at least one sensing element 308. Further, there may be a gap between the elevated structures 318, 320 such that the gas 312 passes between the at least one heating element 306 and the at least one sensing element 308. The description related to the respective elevated structures 318, 320 will be described in conjunction with FIG. 3B.

In one example embodiment, the first portion 302 and the second portion 304 may be made from one or more materials. The one or more materials may comprise at least one of silicon, or glass. Further, the first portion 302 may be coupled with the second portion 304 via an adhesive such that the gap 314 is provided between the first portion 302 and the second portion 304. In one example embodiment, the adhesive may comprise at least one of a frit bond, an anodic bond, an epoxy adhesive, and a eutectic bond. In one example, silicon to silicon bonds may be made with the frit bond and the eutectic bond. In another example, glass to silicon bonds may be made with the anodic bond and the frit bond. In yet another example, each of the glass and silicon may be bonded to silicon with an adhesive such as a silicone, the epoxy adhesive, or cyanoacrylate.

In some embodiments, the at least one heating element 306 positioned beside the at least one sensing element 308 with the gas 312, may produce a significant sensitivity improvement in the thermal conductivity sensor 300 in terms of detecting the thermal conductivity of the gas 312. In some embodiments, the thermal conductivity sensor 300 may rely upon the use of heat and heat transfer. Further, a temperature difference may exist between the at least one heating element 306 and the at least one sensing element 308 in the gas 312 and the change in the heat transfer through the gas 312 to which the at least one heating element 306 and the at least one sensing element 308 are exposed to. Further, the change in the heat transfer may correspond to the change in the thermal property of the gas 312, such as going from the gas 312 to gas 312 plus hydrogen. The change in the heat transfer may change an output of the thermal conductivity sensor 300 and may be directly related to the composition of the gas 312.

In some embodiments, the at least one heating element 306 may be made from a group of materials. In one example, the group of materials may comprise at least one of a Permalloy (81:19 Iron-Nickel (NiFe)) and 60:40 NiFe, preferably. The thin film of Permalloy may have temperature coefficients of resistance between 3600 parts per million per degree Celsius (ppm/° C) and 4100 ppm/° C. In another example, the group of materials may comprise at least one of Platinum (Pt), Chromium (Cr), doped Silicon (Si) or Polysilicon, Nichrome (NiCr), Nickel (Ni), Platinum Silicide (PtSi) and other metal silicides, Tungsten (W), TiN, Aluminum Nitride (AlN), Tungsten Nitride (WN), or any combination thereof.

In some embodiments, the at least one sensing element 308 may be configured to measure a change in temperature of the gas 312 to detect a presence of the gas 312 having a higher thermal conductivity. In one example, the gas 312 may correspond to a hydrogen gas. Further, the at least one sensing element 308 may be configured to measure the change in temperature of the gas 312 to monitor heat transfer within the gas 312 around the thermal conductivity sensor 300. In one embodiment, a change in the heat transfer within the gas 312 around the thermal conductivity sensor 300 may correspond to a change in a thermal property of the gas 312. In some embodiments, the at least one sensing element 308 may comprise at least one of a resistor, a diode, or a thermopile. In some embodiments, the at least one sensing element 308 may be made from a group of materials. In one example, the group of materials may comprise at least one of Chromium (Cr) and Permalloy (80/20 NiFe), and Cr and 60/40 NiFe, preferably. In another example, the group of materials may comprise at least one of polysilicon and aluminum (Al); n-type polysilicon and p-type polysilicon; Ni-Fe alloy and Chromium Disilicide; Chromium Nitride and Copper (Cu); Chromium Nitride and Al; Chromium Nitride and p-type polysilicon; and Copper (Cu) and Cu-Ni alloy. In one example embodiment, the resistor may be made from the group of materials described above. In another example embodiment, the resistor may be made from the group of materials such as Pt, NiCr, PtSi, other metal silicides, W, and other materials known in the art. Furthermore, the diode may correspond either to a p-n junction or a metal-semiconductor.

Further, the thermal conductivity sensor 300 may comprise an ambient temperature sensing element 316 on the first portion 302. The ambient temperature sensing element 316 may be configured to measure the temperature of the gas 312 passing through the gap 314 in a real time. In one example, the ambient temperature sensing element 316 and the at least one heating element 306 may be positioned adjacent to each other. In another example, the ambient temperature sensing element 316 and the at least one sensing element 308 may be positioned adjacent to each other. In some embodiments, the ambient temperature sensing element 316 may comprise at least one of a temperature sensor, or a temperature diode. The at least one sensing element 308 and the ambient temperature sensing element 316 may be positioned in one or more combinations. The one or more combinations may comprise at least one of the temperature resistor and the resistor, the temperature diode and the diode, or the temperature resistor and the thermopile.

FIG. 3B illustrates a top view of the thermal conductivity sensor 300 in the horizontal orientation in accordance with an example embodiment of the present disclosure. FIG. 3B is described in conjunction with FIG. 3A.

As described in FIG. 3A, the at least one heating element 306 and the at least one sensing element 308 may be positioned on respective elevated structures separated via the gap 314 such that the gas 312 passes underneath the at least one heating element 306 and the at least one sensing element 308. The thermal conductivity sensor 300 may comprise an elevated structure 318, and an elevated structure 320. The at least one heating element 306 may be positioned on the elevated structure 318 separated via the gap 314 such that the gas 312 passes underneath the at least one heating element 306. The at least one sensing element 308 may be positioned on the elevated structure 320 separated via the gap 314 such that the gas 312 passes underneath the at least one sensing element 308. In some embodiments, the elevated structure 318, and the elevated structure 320 may separate the at least one heating element 306 from the at least one sensing element 308 such that that the gas 312 under analysis is in between the at least one heating element 306 and the at least one sensing element 308. As a result, the at least one sensing element 308 may not be heated by the at least one heating element 306 to an elevated temperature of the at least one heating element 306, and instead, directly heated by the gas 312.

In some embodiments, the thermal conductivity sensor 300 may operate in a temperature range. The temperature range may correspond to a range of -25 degrees Celsius (° C) to 85 ° C. Further, the thermal conductivity sensor 300 may operate in a pressure range. The pressure range may correspond to a range of sea level to 12,000 feet (ft.) above the sea level. It will be apparent to one skilled in the art that the above-mentioned components of the thermal conductivity sensor 300 have been provided only for illustration purposes, without departing from the scope of the disclosure.

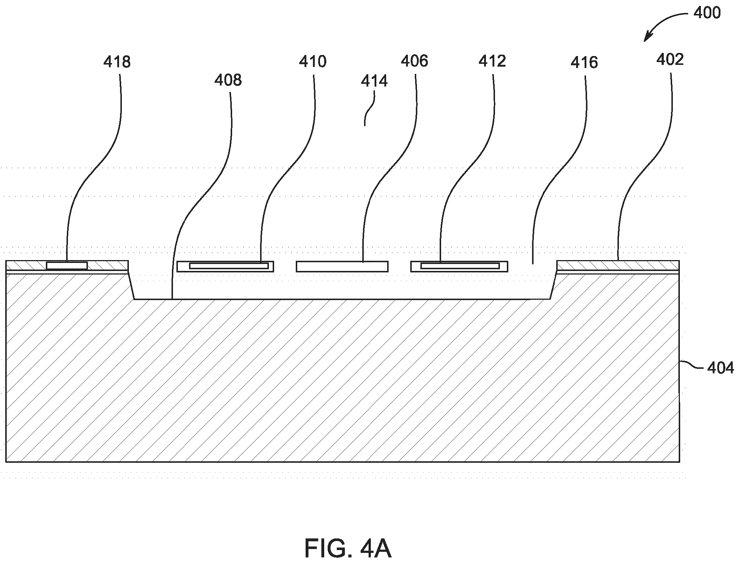

FIG. 4A illustrates a schematic view of another thermal conductivity sensor 400 in a horizontal orientation in accordance with an example embodiment of the present disclosure. The thermal conductivity sensor 400 may comprise a first portion 402 and a second portion 404.

In some embodiments, the first portion 402 may comprise at least one heating element 406, and at least one sensing element. The first portion 402 may be placed on the second portion 404 having a buried cavity 408. The at least one sensing element may comprise a first sensing element 410 and a second sensing element 412. In one example, the second portion 404 may correspond to a base. In some embodiments, the at least one heating element 406 and the at least one sensing element may be positioned such that the at least one heating element 406 and the at least one sensing element are separated by a gap 416 inside the buried cavity 408. Further, the at least one heating element 406 and the at least one sensing element may be positioned such that the at least one heating element 406 is in between the first sensing element 410 and the second sensing element 412. The gap 416 may be configured to allow gas 414 to pass through the gap 416 such that the gas 414 passes around the first sensing element 410, the at least one heating element 406, and the second sensing element 412. In some embodiments, the first sensing element 410, the at least one heating element 406, and the second sensing element 412 may be separated from each other such that that the gas 414 under analysis is in among the first sensing element 410, the at least one heating element 406, and the second sensing element 412. As a result, the first sensing element 410 and the second sensing element 412 may not be heated by the at least one heating element 406 to an elevated temperature of the at least one heating element 406, and instead, directly heated by the gas 414.

In some embodiments, the at least one heating element 406 and the at least one sensing element of the thermal conductivity sensor 400 may be arranged in a plurality of orientations. Further, the plurality of orientations may comprise at least the horizontal orientation. In some embodiments, in the horizontal orientation, the at least one heating element 406 may be positioned between the first sensing element 410 and the second sensing element 412. Further, the at least one heating element 406, the first sensing element 410, and the second sensing element 412 may be positioned on respective elevated structures (not labelled) on the first portion 402, separated via the gap 416 such that the gas 414 passes around the first sensing element 410, the at least one heating element 406, and the second sensing element 412. In one example, the gas 414 passes around the first sensing element 410, the at least one heating element 406, and the second sensing element 412 may correspond to that the gas 414 passes above or below the first sensing element 410, the at least one heating element 406, and the second sensing element 412. Further, there may be a gap between the elevated structures such that gas 414 passes between the at least one heating element 406, the first sensing element 410, and the second sensing element 412.

In some embodiments, the at least one heating element 406 positioned between the first sensing element 410 and the second sensing element 412 and with the gas 414, may produce a significant sensitivity improvement in the thermal conductivity sensor 400 in terms of detecting the thermal conductivity. In some embodiments, the thermal conductivity sensor 400 may rely upon the use of heat and heat transfer. Further, a temperature difference may exist among the first sensing element 410, the at least one heating element 406, and the second sensing element 412 in the gas 414 and the change in the heat transfer through the gas 414 to which the first sensing element 410, the at least one heating element 406, and the second sensing element 412 are exposed to. Further, the change in the heat transfer may correspond to the change in the thermal property of the gas in the gas 414, such as going from the gas 414 to gas 414 plus hydrogen. The change in the heat transfer may change an output of the thermal conductivity sensor 400 and may be directly related to the composition of the gas 414.

In some embodiments, the at least one heating element 406 may be made from a group of materials. In one example, the group of materials may comprise at least one of a Permalloy (81:19 Iron-Nickel (NiFe)) and 60:40 NiFe, preferably. The thin film of Permalloy may have temperature coefficients of resistance between 3600 parts per million per degree Celsius (ppm/° C) and 4100 ppm/° C. In another example, the group of materials may comprise at least one of Platinum (Pt), Chromium (Cr), doped Silicon (Si) or Polysilicon, Nichrome (NiCr), Nickel (Ni), Platinum Silicide (PtSi) and other metal silicides, Tungsten (W), TiN, Aluminum Nitride (AlN), Tungsten Nitride (WN), or any combination thereof.

In some embodiments, the first portion 402 may comprise the at least one sensing element having the first sensing element 410 and the second sensing element 412. The at least one sensing element may be configured to measure a change in temperature of the gas 414 to detect a presence of the gas 414 having a higher thermal conductivity. In one example, the gas 414 may correspond to a hydrogen-containing gas. Further, the at least one sensing element may be configured to measure the change in temperature of the gas 414 to monitor heat transfer within the gas 414 around the thermal conductivity sensor 400. In one embodiment, a change in the heat transfer within the gas 414 around the thermal conductivity sensor 400 may correspond to a change in a thermal property of the gas. In some embodiments, the at least one sensing element may comprise at least one of a resistor, a diode, or a thermopile. In some embodiments, the at least one sensing element may be made from a group of materials. In one example, the group of materials may comprise at least one of Chromium (Cr) and Permalloy (80/20 NiFe), and Cr and 60/40 NiFe, preferably. In another example, the group of materials may comprise at least one of polysilicon and aluminum (Al); n-type polysilicon and p-type polysilicon; Ni-Fe alloy and Chromium Disilicide; Chromium Nitride and Copper (Cu); Chromium Nitride and Al; Chromium Nitride and p-type polysilicon; and Copper (Cu) and Cu-Ni alloy. In one example embodiment, the resistor may be made from the group of materials described above. In another example embodiment, the resistor may be made from the group of materials such as Pt, NiCr, PtSi, other metal silicides, W, and other materials known in the art. Furthermore, the diode may correspond either to a p-n junction or a metal-semiconductor.

Further, the thermal conductivity sensor 400 may comprise an ambient temperature sensing element 418 on the first portion 402. The ambient temperature sensing element 418 may be configured to measure the temperature of the gas 414 passing through the gap 416 in a real time. In one example, the ambient temperature sensing element 418 and the first sensing element 410 may be positioned adjacent to each other. In another example, the ambient temperature sensing element 418 and the second sensing element 412 may be positioned adjacent to each other. In some embodiments, the ambient temperature sensing element 418 may comprise at least one of a temperature sensor, or a temperature diode. The first sensing element 410 and the ambient temperature sensing element 418, or the second sensing element 412 and the ambient temperature sensing element 418 may be positioned in one or more combinations. The one or more combinations may comprise at least one of the temperature resistor and the resistor, the temperature diode and the diode, or the temperature resistor and the thermopile.

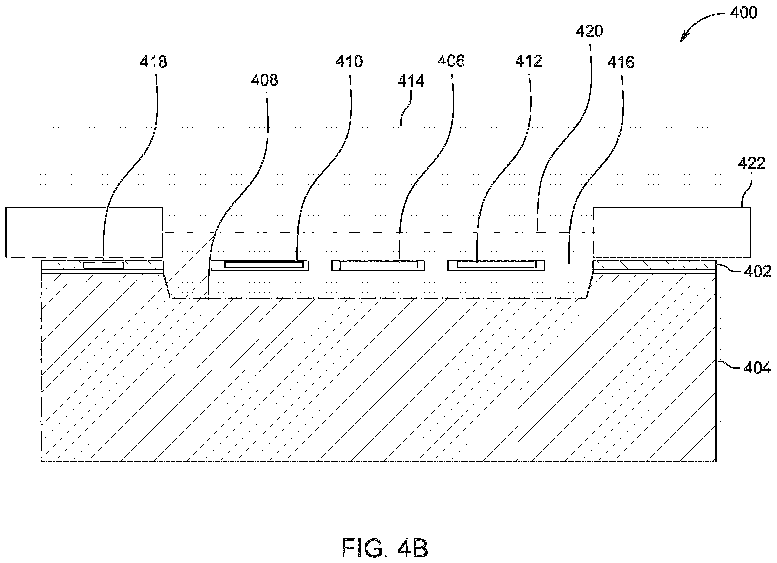

FIG. 4B illustrates a filter media 420 associated with the another thermal conductivity sensor 400 in the horizontal orientation in accordance with an example embodiment of the present disclosure. FIG. 4B is described in conjunction with FIG. 4A.

In some embodiments, the first portion 402 may comprise the at least one heating element 406, and the at least one sensing element. The first portion 402 may be placed on the second portion 404 having the buried cavity 408. Further, at least one package 422 having the filter media 420 may be placed on the first portion 402. The at least one package 422 may correspond to a place where the filter media 420 exists. The filter media 420 may form a layer above the first portion 402. The filter media 420 may be configured to allow the gas 414 to diffuse evenly throughout the gap 416. The filter media 420 may be configured to allow the gas 414 to diffuse evenly such that there is less change in temperature in an instance in which the gas 414 passes through the first sensing element 410, the at least one heating element 406, and the second sensing element 412. The filter media 420 may provide minimal change in the temperature as the gas 414 diffuses evenly among the first sensing element 410, the at least one heating element 406, and the second sensing element 412, thereby optimizing performance and accuracy of the thermal conductivity sensor 400.

In some embodiments, the thermal conductivity sensor 400 may operate in a temperature range. The temperature range may correspond to a range of -25 degrees Celsius (° C) to 85 ° C. Further, the thermal conductivity sensor 400 may operate in a pressure range. The pressure range may correspond to a range pf sea level to 12,000 feet (ft.) above the sea level. It will be apparent to one skilled in the art that the above-mentioned components of the thermal conductivity sensor 400 have been provided only for illustration purposes, without departing from the scope of the disclosure.

In some embodiments, the thermal conductivity sensor 100, the thermal conductivity sensor 300 and the thermal conductivity sensor 400 may hold the same functionality without departing from the scope of the disclosure.

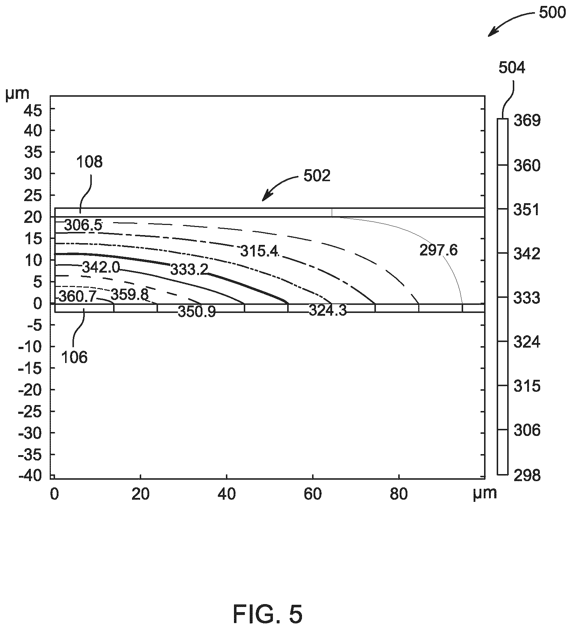

FIG. 5 illustrates a graphical representation 500 of a simulation of the thermal conductivity sensor 100 in the vertical orientation in accordance with an example embodiment of the present disclosure. FIG. 5 is described in conjunction with FIG. 1.

In some embodiments, the graphical representation 500 may represent the transfer of heat by the at least one heating element 106 on the at least one sensing element 108, as illustrated by 502. The x-axis and the y-axis of the graphical representation 500 may represent distance between the at least one heating element 106 and at least one sensing element 108, in microns (µm). In one example, the at least one heating element 106 radiates heat having a temperature in the range of 298 kelvin (K) - 369 K, as illustrated by a temperature scale 504. As the distance between the at least one sensing element 108 increases from the at least one heating element 106, the temperature decreases.

In some embodiments, the heat radiated from the at least one heating element 106 reduces across a vertical direction of the at least one heating element 106 and across a horizontal direction of the at least one heating element 106 in different degrees. In one example, temperature of gas in vertical direction of the at least one heating element 106 at a distance of 15-20 µm ranges between 320 - 297.6 K. In another example, temperature of gas in the horizontal direction of the at least one heating element 106 at the same distance of 15 - 20 µm range between 360.7 - 359.8 K. In some embodiments, the distance of the at least one sensing element 108 from the at least one heating element 106 may be 20 µm, and is exposed to less temperature between 300 K and 306.5 K, as a result, the at least one heating element 106 does not have any effect on the at least one sensing element.

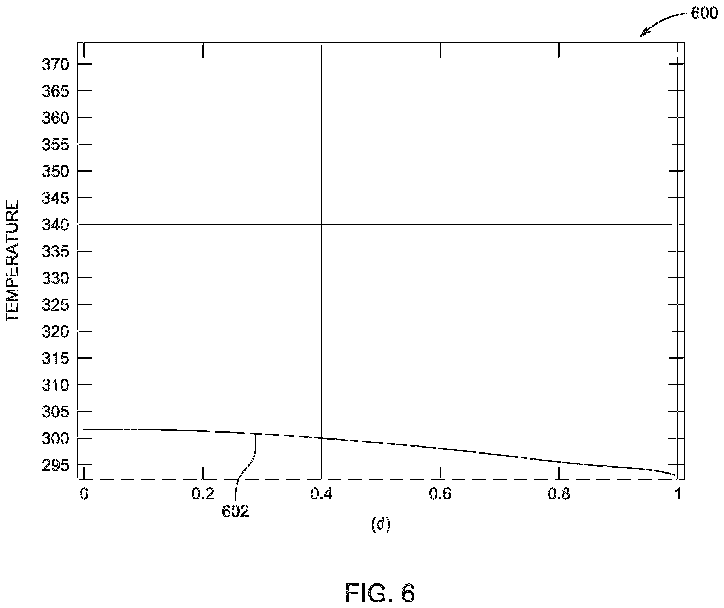

FIG. 6 illustrates a graphical representation 600 of effect of temperature on at least one sensing element in accordance with an example embodiment of the present disclosure. FIG. 6 is described in conjunction with FIG. 1.

In some embodiments, the graphical representation 600 may represent the effect of temperature on the at least one sensing element 108, as illustrated by 602. The x-axis of the graphical representation 600 may represent distance from center of the at least one heating element 106 to the edge of the first portion 102 vertically, in percentage. The y-axis of the graphical representation 600 may represent the temperature of the heat by at least one heating element 106 in K. In one example, when the at least one sensing element 108 is positioned over the at least one heating element 106 by a distance of 20 µm, the at least one sensing element 108 is exposed to a temperature between 300 K and 305 K. Further, as the distance of the at least one sensing element 108 increases with respect to the at least one heating element 106, the temperature evenly drops from 300K to below 295K.

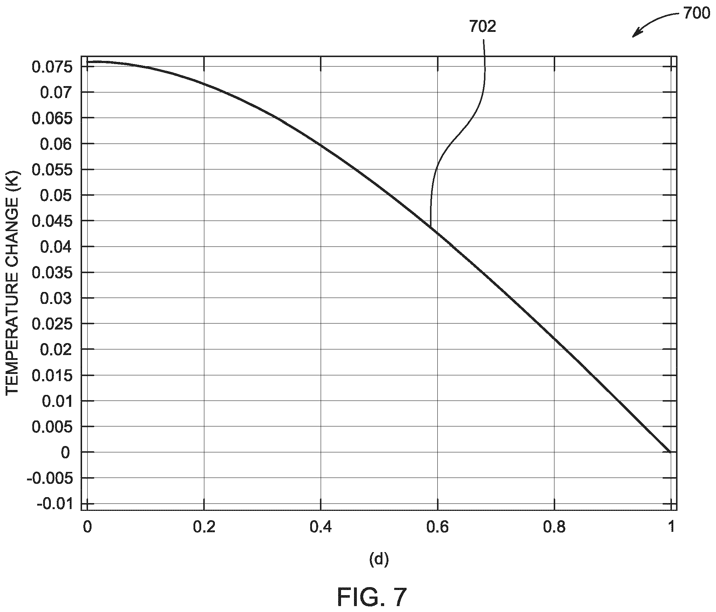

FIG. 7 illustrates a graphical representation 700 of a change in sensing temperature corresponding to 1% change in thermal conductivity of the gas in accordance with an example embodiment of the present disclosure. FIG. 7 is described in conjunction with FIG. 1.

In some embodiments, the graphical representation 700 may represent a change in sensing temperature corresponding to 1% change in thermal conductivity of the gas, as illustrated by 702. The x-axis of the graphical representation 700 may represent distance from center of the at least one heating element 106 to the edge of the first portion 102 vertically. The y-axis of the graphical representation 700 may represent the change in sensing temperature. In one example, as the distance from center of the at least one heating element 106 to the edge of the first portion 102 decreases, the change in sensing temperature increases.

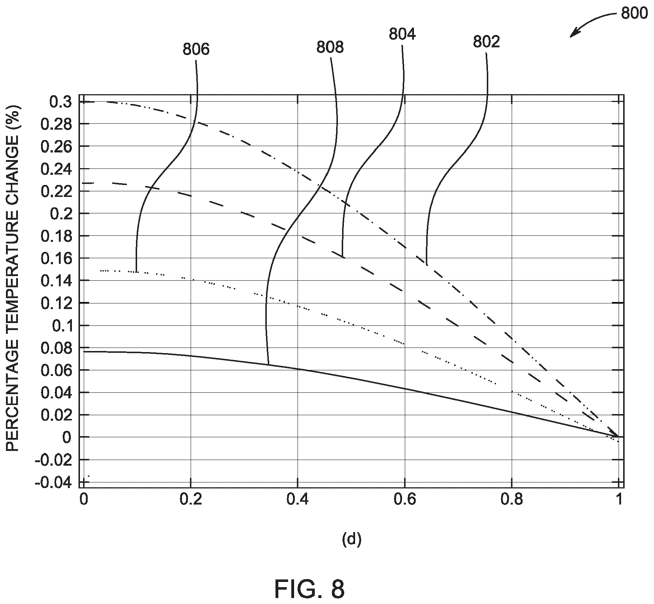

FIG. 8 illustrates a graphical representation 800 of a parallel plate preliminary simulation of the changes in sensing temperature due to changes in thermal conductivity sensor 100 in accordance with an example embodiment of the present disclosure.

In some embodiments, the graphical representation 800 may represent a parallel plate preliminary simulation of the changes in sensing temperature of thermal conductivity sensor 100 with varying thermal conductivity of gas, as illustrated by curve 802, curve 804, curve 806, and curve 808. The x-axis of the graphical representation 800 may represent distance from center of the at least one heating element 106 to the edge of the first portion 102 in vertical direction. The y-axis of the graphical representation 800 may represent the change in sensing temperature (i.e., percentage temperature change (%)). Further, the thermal conductivity denoted by "k" that is derived from multiplying a value with the thermal conductivity of the gas denoted by "kₒ". The value may correspond to concentration of a high thermal conductivity gas specie in the gas. In one example k = 1.01 kₒ, as illustrated by the curve 808. In another example k = 1.02 kₒ, as illustrated by the curve 806. In yet another example k = 1.03 kₒ, as illustrated by the curve 804. In one example k = 1.04 kₒ, as illustrated by the curve 802. As concentration of the high thermal conductivity gas specie increases, the thermal conductivity of the gas mixture increases.

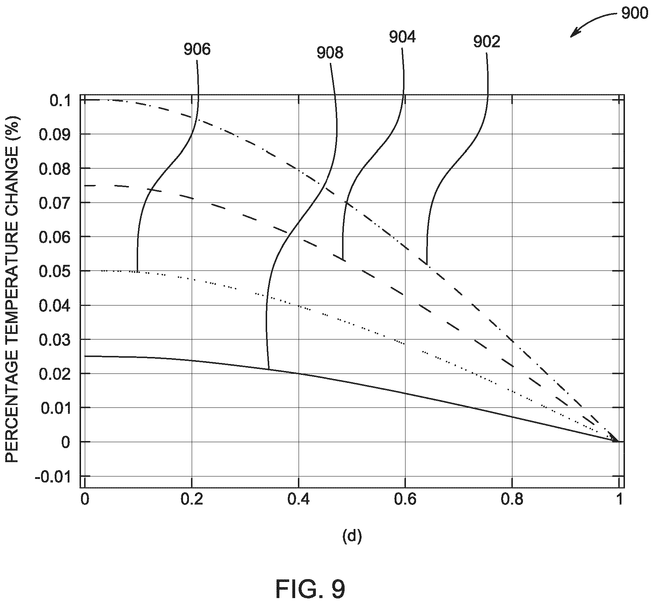

FIG. 9 illustrates a graphical representation 900 of another parallel plate preliminary simulation of the thermal conductivity sensor 100 in accordance with an example embodiment of the present disclosure.

In some embodiments, the graphical representation 900 may represent another parallel plate preliminary simulation of the thermal conductivity sensor 100 with varying thermal conductivity, as illustrated by curve 902, curve 904, curve 906, and curve 908. The x-axis of the graphical representation 900 may represent distance from center of the at least one heating element 106 to the edge of the first portion 102 in vertical direction. The y-axis of the graphical representation 900 may represent the percentage change in sensing temperature (i.e., percentage temperature change (%)) corresponding to thermal conductivity change of gas mixture. Further, the thermal conductivity denoted by "k" that is derived from multiplying a value with the thermal conductivity of the gas denoted by "kₒ". The value may correspond to concentration of the gas. In one example k = 1.01 kₒ, as illustrated by the curve 908. In another example k = 1.02 kₒ, as illustrated by curve 906. In yet another example k = 1.03 kₒ, as illustrated by curve 904. In one example k = 1.04 kₒ, as illustrated by the curve 902. As concentration of the high thermal conductivity gas specie increases, the thermal conductivity of the gas mixture increases.

In some embodiments, a method of the thermal conductivity sensor 100 is disclosed. The method may comprise positioning the first portion 102 of the thermal conductivity sensor 100, having at least one heating element 106 and the second portion 104 of the thermal conductivity sensor 100, having at least one sensing element 108, such that the at least one heating element 106 and the at least one sensing element 108 are separated by the gas channel or the gap between the first portion 102 and the second portion 104 that is configured to allow the gas to pass through the gas channel or the gap. Further, the at least one sensing element 108 may be configured to measure the change in temperature of the gas to detect the presence of the gas having the higher thermal conductivity.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A thermal conductivity sensor comprising:
a first portion having at least one heating element; and
a second portion having at least one sensing element,
wherein the first portion and the second portion are positioned such that the at least one heating element and the at least one sensing element are separated by a gas channel or gap between the first portion and the second portion that is configured to allow gas to pass through the gas channel or gap such that the gas passes between the at least one heating element and the at least one sensing element; and
wherein the at least one sensing element is configured to measure a change in temperature of the gas to detect a presence of the gas having a higher thermal conductivity.

2. The thermal conductivity sensor of claim 1, wherein the at least one sensing element is configured to measure the change in temperature of the gas to monitor heat transfer within the gas around the thermal conductivity sensor.

3. The thermal conductivity sensor of claim 2, wherein a change in the heat transfer within the gas around the thermal conductivity sensor corresponds to a change in a thermal property of the gas.

4. The thermal conductivity sensor of claim 1, wherein the first portion and the second portion of the thermal conductivity sensor are arranged in a plurality of orientations, wherein the plurality of orientations comprise at least one of a vertical orientation and a horizontal orientation.

5. The thermal conductivity sensor of claim 4, wherein in the vertical orientation, the at least one heating element is positioned over or under the at least one sensing element and separated via the gas channel such that the gas passes around the at least one heating element and the at least one sensing element.

6. The thermal conductivity sensor of claim 4, wherein in the horizontal orientation, the at least one heating element is positioned beside the at least one sensing element and the at least one heating element and the at least one sensing element are positioned on respective elevated structures separated via the gap such that the gas passes around the at least one heating element and the at least one sensing element.

7. The thermal conductivity sensor of claim 4, wherein in the vertical orientation, the first portion corresponds to a top cap and the second portion corresponds to a base.

8. The thermal conductivity sensor of claim 7, wherein each end of the top cap is coupled with a corresponding end of the base via an adhesive such that the gas channel is provided between the top cap and the base.

9. The thermal conductivity sensor of claim 7, wherein the top cap comprises a set of vents, wherein the set of vents are configured to allow diffusion of the gas into and through the gas channel.

10. The thermal conductivity sensor of claim 1, further comprising an ambient temperature sensing element configured to measure the temperature of the gas passing through the gas channel in a real time.

11. A method comprises:
positioning a first portion of a thermal conductivity sensor, having at least one heating element and a second portion of the thermal conductivity sensor having at least one sensing element, such that the at least one heating element and the at least one sensing element are separated by a gas channel or gap between the first portion and the second portion that is configured to allow gas to pass through the gas channel or gap such that the gas passes between the at least one heating element and the at least one sensing element, and
wherein the at least one sensing element is configured to measure a change in temperature of the gas to detect a presence of the gas having a higher thermal conductivity.

12. The method of claim 11, wherein the first portion and the second portion of the thermal conductivity sensor are arranged in a plurality of orientations, wherein the plurality of orientation comprises at least one of a vertical orientation and a horizontal orientation.

13. The method of claim 12, wherein in the vertical orientation, the at least one heating element is positioned over or under the at least one sensing element and separated via the gas channel such that the gas passes around the at least one heating element and the at least one sensing element.

14. The method of claim 12, wherein in the horizontal orientation, the at least one heating element is positioned beside the at least one sensing element and the at least one heating element and the at least one sensing element are positioned on respective elevated structures separated via the gap such that the gas passes around the at least one heating element and the at least one sensing element.

15. The method of claim 11, wherein the at least one sensing element is configured to measure the change in temperature of the gas to monitor heat transfer within the gas around the thermal conductivity sensor.
